# EUROPEAN PATENT APPLICATION

(11) **EP 1 947 096 A1**
(43) Date of publication of application: **23.07.2008**
(21) Application number: 06822951.7
(22) Date of filing: 27.10.2006
(51) Int. Cl.: C07D 333/56, C07D 333/68, C07D 409/06, C07D 409/14, C07D 471/04, C07B 61/00

(54) **3-HYDROXYMETHYLBENZO[b]THIOPHENE DERIVATIVE AND METHOD FOR PRODUCING SAME**

(30) Priority: 27.10.2005 JP 2005313032
(71) Applicant: Teijin Pharma Limited, Chiyoda-ku, Tokyo 100-0013 (JP)
(72) Inventor: YAJIMA, Naoki, Iwakuni-shi, Yamaguchi, 740-0014 (JP); HIROKI, Yasuhiro, Chiba, 261-0003 (JP); YOSHINO, Hiroshi, Chiba, 272-0823 (JP); KOIZUMI, Tatsuya, Chiba, 275-0016 (JP)
(74) Representative: Hallybone, Huw George
(86) International application number: PCT/JP2006/322033
(87) International publication number: WO 2007/049812

(57) **Abstract**

The invention provides 3-hydroxymethylbenzo[b]thiophene derivatives useful as production intermediates for chymase inhibitors, and a process for their production.

The invention relates to 3-hydroxymethylbenzo[b]thiophene derivatives represented by the following formula (II), and a process for their production.

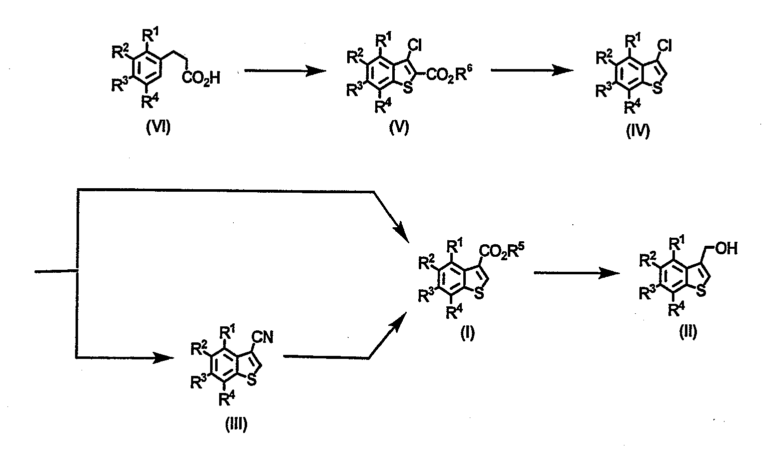

## Description

### Technical Field

The present invention relates to 3-hydroxymethylbenzo[b]thiophene derivatives that are important as production intermediates for compounds useful as drugs, and to a process for their production. More specifically, it relates to a process for production of production intermediates that are useful for synthesis of compounds having chymase inhibiting activity in the body and that can be used as prophylactic or therapeutic agents for inflammatory diseases, allergic diseases, respiratory diseases, circulatory diseases or bone/cartilage metabolic disorders.

### Background Art

Formula (II) 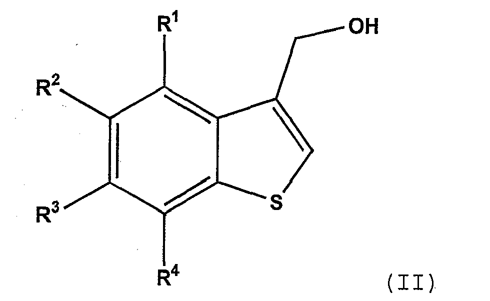 wherein:
R¹ represents C1-6 alkyl, C1-6 halogenated alkyl, a halogen atom, cyano, C1-6 alkoxy, C1-6 alkylthio, C1-6 acyloxy, C1-6 acylamino or C1-6 halogenated alkoxy; and
R², R³ and R⁴ may simultaneously or each independently represent a hydrogen atom, C1-6 alkyl, C1-6 halogenated alkyl, a halogen atom, cyano, C1-6 alkoxy, C1-6 alkylthio, C1-6 acyloxy, C1-6 acylamino group or C1-6 halogenated alkoxy.

The 3-hydroxymethylbenzo[b]thiophene derivatives represented by formula (II) above are of great importance as intermediates in the production of pharmacologically active compounds.

For example, compounds wherein the hydroxyl group in compounds represented by formula (II) above is substituted with bromine can serve as synthetic intermediates for the benzimidazole derivatives disclosed in International Patent Publication WO 01/53291, and are very important as intermediates for production of pharmacologically active compounds. These benzimidazole derivatives have chymase inhibiting activity in the body and are promising as compounds with applications as prophylactic or therapeutic agents for inflammatory diseases, allergic diseases, respiratory diseases, circulatory diseases, or bone/cartilage metabolic disorders.

So far, it has so far been extremely difficult to produce benzothiophene derivatives having a hydroxymethyl group at the 3-position and regioselective placement of other substituents, as in the compounds represented by formula (II) above, and their industrial production has not been feasible. For example, existing processes synthesize 3-formyl-benzo[b]thiophenes by Vilsmeier reaction of benzo[b]thiophenes (J. Org. Chem., 72, 1422, (1957)) followed by their reduction, or synthesize 3-trichloroacetyl-benzo[b]thiophene derivative starting materials by Friedel-Crafts reaction of benzo [b] thiophenes (J. Chem. Soc., Perkin Trans. 2, 1250, (1973)) followed by their hydrolysis and subsequent reduction. However, in all such processes, substitution reaction proceeds either at positions 2 and 3 or at any position(s) of positions 2 to 7 on the benzo[b]thiophene ring, depending on the type and positions of the substituents that are initially present. The selectivity is not very high and tends to depend on the substrate used and the reaction conditions. Also, it is extremely difficult to isolate only the target compound from the resulting mixture.

International Patent Publication WO 02/066457 and International Patent Publication WO 02/090345 disclose examples of production processes for obtaining benzothiophene derivatives having a hydroxymethyl group at the 3-position and regioselective placement of other substituents, similar to the compounds represented by formula (II) above. However, production processes with higher yields than the process described in International Patent Publication WO 02/066457 and with shorter steps than the process described in International Patent Publication WO 02/090345 are desired. High yields with short production steps are major advantages for large-scale synthesis.

### Disclosure of the Invention

It is an object of the present invention to provide a process suitable for regioselective, high-yield and more rapid production of 3-hydroxymethyl-4-methylbenzo[b]thiophenes which are useful as production intermediates for the chymase inhibiting compounds described in International Patent Publication WO01/53291.

As a result of much diligent research directed toward achieving the aforestated object, the present inventors have discovered a regioselective process for production of 3-hydroxymethylbenzo[b]thiophene derivatives with shorter steps and higher yield than the

### prior art.

Specifically, the invention provides:
(1) A production process in which a compound represented by the following formula (I): 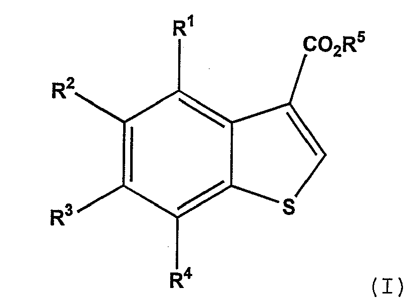 wherein
   R¹ represents C1-6 alkyl, C1-6 halogenated alkyl, a halogen atom, cyano, C1-6 alkoxy, C1-6 alkylthio, C1-6 acyloxy, C1-6 acylamino or C1-6 halogenated alkoxy; and
   R², R³ and R⁴ simultaneously or each independently represent a hydrogen atom, C1-6 alkyl, C1-6 halogenated alkyl, a halogen atom, cyano, C1-6 alkoxy, C1-6 alkylthio, C1-6 acyloxy, C1-6 acylamino or C1-6 halogenated alkoxy; and
   R⁵ represents a hydrogen atom or a C1-6 alkyl group;
   is reduced to obtain a 3-hydroxymethylbenzo[b]thiophene derivative represented by the following formula (II): 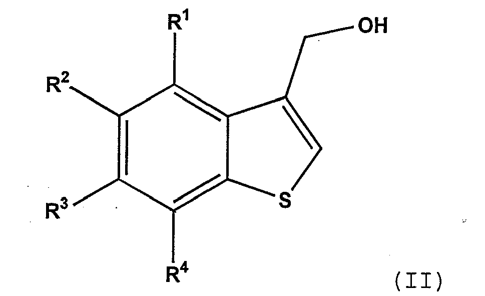 wherein R¹, R², R³ and R⁴ are as defined in formula (I).
(2) The production process according to (1), wherein a metal hydride complex is used as the reducing agent.
(3) The production process according to (2), wherein the metal hydride complex is sodium bis(2-methoxyethoxy)aluminum hydride.
(4) The production process according to any one of (1) to (3), wherein a compound represented by the following formula (III) is subjected to hydrolysis to produce a benzo[b]thiophene derivative represented by formula (I): 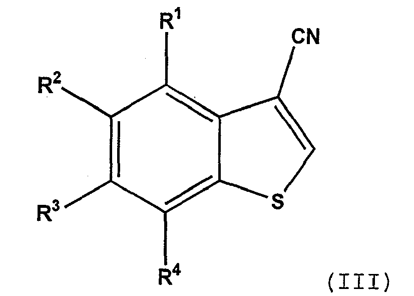 wherein R¹, R², R³ and R⁴ are as defined in formula (I) .
(5) The production process according to (4), wherein the hydrolysis is basic hydrolysis.
(6) The production process according to (5), characterized in that the basic hydrolysis employs sodium hydroxide.
(7) The production process according to any one of (4) to (6), wherein the chloro group of a compound represented by the following formula (IV): 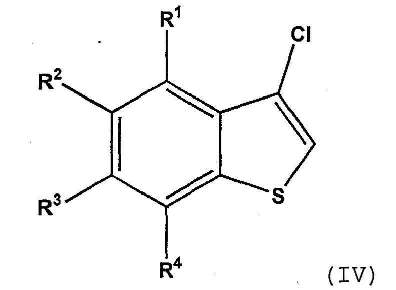 wherein R¹, R², R³ and R⁴ are as defined in formula (I);
   is substituted with a cyano group, to produce the benzo[b]thiophene derivative represented by formula (III).
(8) The production process according to (7), wherein potassium cyanide, sodium cyanide, copper cyanide or zinc cyanide is used to substitute the chloro group of the compound represented by formula (IV) with a cyano group.
(9) The production process according to (8), wherein a nickel catalyst is used.
(10) The production process according to any one of (1) to (3), wherein the chloro group of a compound represented by formula (IV) is substituted with a carboxyl group, to produce a benzo[b]thiophene derivative represented by formula (I).
(11) The production process according to (10), characterized by using carbon monoxide.
(12) The production process according to (11), characterized by using a palladium catalyst.
(13) The production process according to any one of (7) to (12), wherein a benzo[b]thiophene derivative represented by formula (IV) is produced by decarboxylation of a compound represented by the following formula (V): 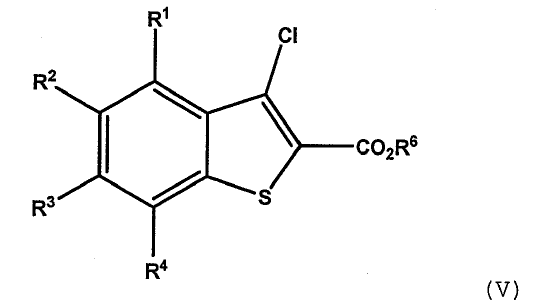 wherein R¹, R², R³ and R⁴ are as defined in formula (I), and R⁶ represents a hydrogen atom or a metal.
(14) The production process according to (13), wherein a benzo[b]thiophene derivative represented by formula (V) is produced by reaction of a compound represented by the following formula (VI): 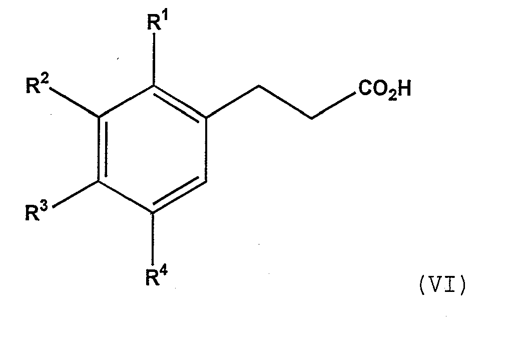 wherein R¹, R², R³ and R⁴ are as defined in formula (I); with thionyl chloride for cyclization.
(15) The production process according to (13) or (14), wherein R⁶ in formula (V) is an alkali metal.
(16) The production process according to (15), wherein R⁶ in formula (V) is a sodium atom.
(17) A production process for benzimidazole derivatives represented by formula (XX), which includes the following production steps:
   (i) a production process for a compound represented by formula (II), according to any one of (1) to (16); and
   (ii) a process in which a benzimidazole derivative represented by formula (XX) is produced from the compound represented by formula (II); 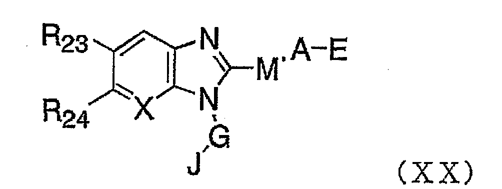 in formula (XX),

   R₂₃ and R₂₄ simultaneously or each independently represent a hydrogen atom, a halogen atom, trihalomethyl, cyano, hydroxyl, C1-4 alkyl or C1-4 alkoxy, or R₂₃ and R₂₄ may together form -O-CH₂O-, -O-CH₂CH₂O- or -CH₂CH₂CH₂- (in which case the carbon atoms may be optionally substituted with one or more C1-4 alkyl groups);
   A represents a substituted or unsubstituted C1-7 straight-chain, cyclic or branched alkylene group or alkenylene group, which may include one or more group(s) selected from the group consisting of -O-, -S-, -SO₂- and -NR₂₅- (where R₂₅ represents a hydrogen atom or a straight-chain or branched C1-6 alkyl group), and the substituent(s) on the groups may be a halogen atom or hydroxyl, nitro, cyano, straight-chain or branched C1-6 alkyl or straight-chain or branched C1-6 alkoxy groups (including cases where two adjacent groups form an acetal bond), straight-chain or branched C1-6 alkylthio, straight-chain or branched C1-6 alkylsulfonyl, straight-chain or branched C1-6 acyl, straight-chain or branched C1-6 acylamino, trihalomethyl, trihalomethoxy, phenyl, oxo or phenoxy optionally substituted with one or more halogen atom(s), and one or more of the substituents at any desired position of the alkylene or alkenylene group may be independently substituted, with the exclusion of cases where M in the formula is a single bond, and carbons of A bonded to M are simultaneously substituted with hydroxyl and phenyl groups;
   E represents -COOR₂₅, -SO₃R₂₅, -CONHR₂₅, -SO₂NHR₂₅, tetrazol-5-yl, 5-oxo-1,2,4-oxadiazol-3-yl or 5-oxo-1,2,4-thiadiazol-3-yl (where R₂₅ is as defined above);
   M represents a single bond or -S(O)m-, where m is an integer of 0-2;
   G and J both represent formula (II) above, with G representing the methylene group at position 3 of the benzothiophene of formula (II), and the hydroxyl group of formula (II) is substituted for the nitrogen atom on the benzimidazole ring; and
   X represents -CH= or a nitrogen atom.
(18) A compound represented by the following formula (I): 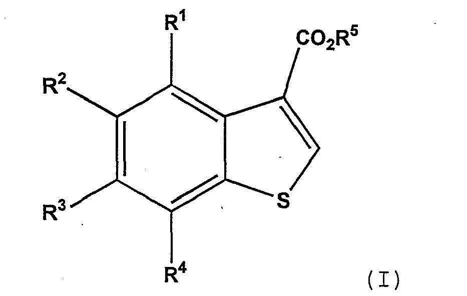 wherein R¹, R², R³, R⁴ and R⁵ are as defined above.
(19) The compound according to (18), wherein R², R³ and R⁴ are hydrogen atoms.
(20) The compound according to (18) or (19) wherein R¹ is a methyl group.
(21) The compound according to any one of (18) to (20), wherein R⁵ is an ethyl group or a hydrogen atom.

The products of the production process of the invention may be formed as salts, and produced salts or salts obtained from the products are also encompassed within the scope of the invention.

The invention allows 3-hydroxymethylbenzo[b]thiophene derivatives that are useful as production intermediates for chymase inhibitor compounds to be produced with short steps in a regioselective manner, and therefore has high industrial value.

### Best Mode for Carrying Out the Invention

The production process for compounds represented by formula (I) is summarized by formula (VII) below: 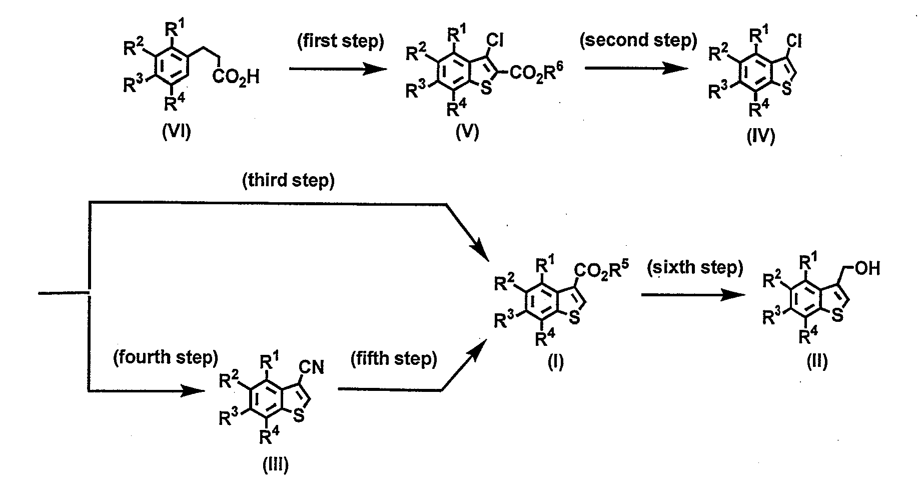 wherein
R¹ represents C1-6 alkyl, C1-6 halogenated alkyl, a halogen atom, cyano, C1-6 alkoxy, C1-6 alkylthio, C1-6 acyloxy, C1-6 acylamino or C1-6 halogenated alkoxy;
R², R³ and R⁴ simultaneously or each independently represent a hydrogen atom, C1-6 alkyl, C1-6 halogenated alkyl, a halogen atom, cyano, C1-6 alkoxy, C1-6 alkylthio, C1-6 acyloxy, C1-6 acylamino or C1-6 halogenated alkoxy, R⁵ represents a hydrogen atom or a C1-6 alkyl group; and
R⁶ represents a hydrogen atom or a metal. The reaction conditions may be selected as appropriate by a person skilled in the art depending on the nature of the substrate used, and there is no strict limitation to the conditions described below.

In the production process of the invention, R¹ is preferably a C1-4 alkyl group, and most preferably R¹ is a methyl group. R², R³ and R⁴ are preferably all hydrogen atoms. R⁵ is preferably a hydrogen atom or an ethyl group. R⁶ is preferably a hydrogen atom or a sodium atom.

As used herein, the term "C1-6 alkyl" refers to a C1-6 straight-chain or branched alkyl group. As examples there may be mentioned methyl, ethyl, n-propyl, n-butyl, n-pentyl, n-hexyl, n-heptyl, n-octyl, isopropyl, isobutyl, sec-butyl, tert-butyl, isopentyl, neopentyl, tert-pentyl, isohexyl, 2-methylpentyl and 1-ethylbutyl.

As used herein, the term "halogen atom" refers to fluorine, chlorine, bromine, iodine and the like, among which fluorine, chlorine and bromine may be mentioned as preferred examples.

As used herein, the term "C1-6 halogenated alkyl" refers to a group comprising a halogen atom and the aforementioned "C1-6 alkyl" group. As examples there may be mentioned fluoromethyl, chloromethyl, bromomethyl, 1-fluoroethyl, 2-fluoroethyl and 3-fluoro-n-propyl.

As used herein, the term "C1-6 alkoxy" refers to a group comprising the aforementioned "C1-6 alkyl" group and an oxy group. As examples there may be mentioned methoxy, ethoxy, isopropoxy and tert-butoxy.

As used herein, the term "C1-6 alkylthio" refers to a group comprising the aforementioned "C1-6 alkyl" group and a thio group. As examples there may be mentioned methylthio and ethylthio.

As used herein, the term "C1-6 acyloxy" refers to a group comprising the aforementioned "C1-6 acyl" group and an oxy group. The term "C1-6 acyl" refers to a combination of the aforementioned "C1-6 alkyl" group and a carbonyl group, examples of which include acetyl, propionyl, butyryl, isobutyryl, valeryl, isovaleryl and pivaloyl. Examples of "C1-6 acyloxy" groups include acetoxy, propionyloxy, butyryloxy, isobutyryloxy, valeryloxy, isovaleryloxy and pivaloyloxy.

As used herein, the term "C1-6 acylamino" refers to a group comprising the aforementioned "C1-6 acyl" group and an amino group. As examples there may be mentioned acetylamino, propionylamino, butyrylamino, isobutyrylamino, valerylamino, isovalerylamino and pivaloylamino.

As used herein, the term "C1-6 halogenated alkoxy" refers to a group comprising a halogen atom and the aforementioned "C1-6 alkyloxy" group. As examples there may be mentioned fluoromethoxy, 2-chloroethoxy, 1-bromoisopropoxy and 2-iodo-tert-butoxy.

### (First step)

This is a step of building a benzo[b]thiophene ring using thionyl chloride, and synthesizing compound (V) from compound (VI). The method described in J. Org. Chem., 41, 3399(1976) may be referred to for the reaction of this step. The optimum conditions for a carboxylic acid according to the invention are 5 equivalents of thionyl chloride and 0.1 equivalent of pyridine. The reaction temperature will normally be 130-170°C and preferably 150-160°C. If the reaction is carried out at 150°C, it will usually go to completion in 3-6 hours. The product contains carboxyl groups and may therefore form a salt. As examples of salts there may be mentioned alkali metal salts or alkaline earth metal salts, such as sodium salt. The reaction is preferably carried out without a solvent from the viewpoint of shortening the reaction time, but a solvent such as chlorobenzene, toluene or N,N-dimethylformamide may be used.

In this step, R¹ in compound (VI) is substituted with a functional group that is not a hydrogen atom, and therefore the cyclization reaction occurs in a regioselective manner.

### (Second step)

This is a step of decarboxylation of the carboxyl group of the cyclized product obtained in the previous step, by reaction to synthesis compound (IV) from compound (V). Copper is usually used at 0.1-1.0 equivalent. The reaction solvent used may be quinoline, N,N-dimethylformamide, N,N-dimethylacetamide or dimethylaniline. Quinoline is the preferred solvent. However, N,N-dimethylformamide is preferred when the carboxylic acid salt of the cyclized product is used as the starting material. The reaction temperature will normally be 130-180°C and preferably 150-160°C. If the reaction is carried out at 150°C, it will usually go to completion in 3-6 hours.

### (Third step)

This is a step of carbonylation wherein the chloro group on the aromatic ring is converted to a carboxylic acid ester group, as a reaction to synthesize compound (I) from compound (IV). As catalysts to be used for the invention there may be mentioned palladium acetate, dibromobis(triphenylphosphine)palladium(II), dichlorobis(triphenylphosphine)palladium(II), dichloro[1,1'-bis(diphenylphosphino)ferrocene]palladium(II) and tetrakis(triphenylphosphine)palladium(0). As ligands there may be used diphenylphosphinoethane, diphenylphosphinopropane, diphenylphosphinobutane, triphenylphosphine and tri-t-butylphosphine. As bases there may be mentioned triethylamine, sodium acetate, sodium ethoxide, silver carbonate and 1,8-diazabicyclo[5.4.0]undec-7-ene. Also, sodium iodide, sodium chloride or sodium bromide may be used as additives. The reaction solvent used may be an alcohol-based solvent such as ethanol or methanol. The carbon monoxide pressure is 0.1-1.0 MPa. Particularly preferred conditions are palladium acetate as the catalyst, diphenylphosphinopropane as the ligand, 1,8-diazabicyclo[5.4.0]undec-7-ene as the base, sodium iodide as an additive, ethanol as the reaction solvent and a carbon monoxide pressure of 0.6 MPa. The reaction temperature is 50°C-150°C and preferably 120°C-140°C. It is more preferably in the vicinity of 130°C.

### (Fourth step)

This is a step in which the chloro group on the aromatic ring is replaced by a cyano group, as a reaction to synthesize compound (III) from compound (IV). The catalyst used for the invention may be nickel bromide, nickel chloride or dibromobis(triphenylphosphine)nickel(II). As ligands there may be used triphenylphosphine, diphenylphosphinoethane, diphenylphosphinopropane, diphenylphosphinobutane, tri-t-butylphosphine and the like. The catalyst will usually be used at 0.03-0.50 equivalent, but it is preferably used at 0.05 equivalent or greater. The reaction solvent used may be ethanol, methanol, tetrahydrofuran, acetonitrile, N,N-dimethylformamide, N,N-dimethylacetamide, dimethylsulfoxide, hexamethyltriamide phosphate, 1,1,3,3-tetramethylurea, sulfolane or the like, among which tetrahydrofuran and ethanol are particularly preferred. As cyanating agents there may be used potassium cyanide, sodium cyanide, copper cyanide and zinc cyanide, among which potassium cyanide is particularly preferred. The reaction temperature is 50°C-150°C and preferably 70°C-100°C. It is more preferably in the vicinity of 90°C.

### (Fifth step)

This is a step of hydrolyzing the cyano group, as a reaction to synthesize compound (I) from compound (III). The hydrolysis in this step is preferably basic hydrolysis. For basic hydrolysis there may be used sodium hydroxide, potassium hydroxide, calcium hydroxide, barium hydroxide or the like, with sodium hydroxide being preferred. The alkali content is preferably 3.0-10 equivalents and most preferably 3.0-5.0 equivalents. The reaction temperature will normally be 80-200°C and is preferably between 160°C and 200°C. If the reaction is carried out at 190°C, it will usually go to completion in 2-3 hours. The reaction solvent used may be an alcohol-based solvent such as methanol or ethanol and ethylene glycol, with ethylene glycol being preferred since the reaction is conducted at high temperature.

### (Sixth step)

This is a step of reducing the carboxylic acid or carboxylic acid ester, as a reaction to synthesize compound (II) from compound (I). As reducing agents there are preferred metal hydride complexes, such as sodium bis(2-methoxyethoxy)aluminum hydride, lithium aluminum hydride, sodium borohydride, borane-tetrahydrofuran complex and the like. Sodium bis(2-methoxyethoxy)aluminum hydride is preferred among these. As solvents there may be mentioned tetrahydrofuran, toluene and the like, although alcohols such as methanol and ethanol may be added for increased reaction efficiency. When sodium borohydride is used, an additive such as calcium chloride may be used. The reaction temperature is between -30°C and 100°C, and preferably between 0°C and 30°C.

The numbers of steps in the two routes for formula (VII) are therefore 4 and 5, respectively. In the examples provided below, the portable yields were 49.7% when using the third step and 45.2% when using the fourth and fifth steps. The yield is higher with the third step because fewer steps are carried out. However, using the fourth and fifth steps helps reduce industrial cost by using a relatively inexpensive nickel catalyst.

A benzothiophene derivative of formula (II) produced by the process described above can be used to synthesize medically useful benzimidazole derivatives (for example, formula (XX)), according to, for example, the method described in International Patent Publication WO 01/53291, 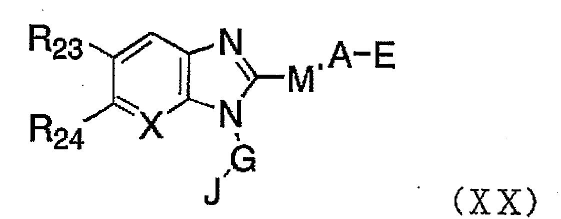 in formula (XX),
R₂₃ and R₂₄ simultaneously or each independently represent a hydrogen atom, a halogen atom, trihalomethyl, cyano, hydroxyl, C1-4 alkyl or C1-4 alkoxy, or R₂₃ and R₂₄ may together form -O-CH₂-O-, -O-CH₂CH₂-O- or -CH₂CH₂CH₂- (in which case the carbon atoms may be optionally substituted with one or more C1-4 alkyl groups);
A represents a substituted or unsubstituted C1-7 straight-chain, cyclic or branched alkylene group or alkenylene group, which may include one or more group(s) selected from the group consisting of -O-, -S-, -SO₂- and -NR₂₅- (where R₂₅ represents a hydrogen atom or a straight-chain or branched C1-6 alkyl group), and the substituent(s) on the groups may be a halogen atom or hydroxyl, nitro, cyano, straight-chain or branched C1-6 alkyl or straight-chain or branched C1-6 alkoxy groups (including cases where two adjacent groups form an acetal bond), straight-chain or branched C1-6 alkylthio, straight-chain or branched C1-6 alkylsulfonyl, straight-chain or branched C1-6 acyl, straight-chain or branched C1-6 acylamino, trihalomethyl, trihalomethoxy, phenyl, oxo or phenoxy optionally substituted with one or more halogen atom(s), and one or more of the substituents at any desired position of the alkylene or alkenylene group may be independently substituted, with the exclusion of cases where M in the formula is a single bond and hydroxyl and phenyl groups simultaneously substitute carbons of A bonded to M;
E represents -COOR₂₅, -SO₃R₂₅, -CONHR₂₅, -SO₂NHR₂₅, tetrazol-5-yl, 5-oxo-1,2,4-oxadiazol-3-yl or 5-oxo-1,2,4-thiadiazol-3-yl (where R₂₅ is as defined above);
M represents a single bond or -S(O)m-, where m is an integer of 0-2;
G and J together represent formula (II) above, with G representing the methylene group at position 3 of the benzothiophene of formula (II), and the hydroxyl group of formula (II) being substituted by the nitrogen atom on the benzimidazole ring; and
X represents -CH= or a nitrogen atom.

When E is COOR₂₅ and M is S in the benzimidazole derivative (XX), production may be carried out by Synthesis Scheme (A) or Synthesis Scheme (B) shown below. Synthesis Scheme (A): 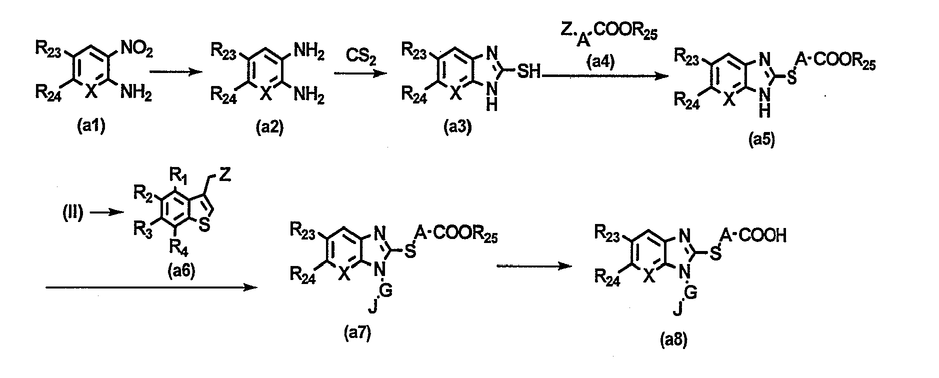 wherein Z represents a halogen, sulfonyloxy or an ammonium salt, and R₂₃, R₂₄, R₂₅, A, G, J and X are as defined above.

Specifically, the nitro group of a 2-nitroaniline derivative (a1) is reduced to obtain an orthophenylenediamine (a2). This is reacted with CS₂ to form compound (a3), after which a halide ester derivative (a4) is reacted therewith to obtain (a5) which is reacted with a halide derivative (a6) obtained by halogenating the hydroxyl group of formula (II), to obtain compound (a7). If necessary, this product may be further subjected to hydrolysis to obtain a benzimidazole derivative (a8) where R₂₅ is a hydrogen atom.

Reduction of the nitro group may be accomplished under ordinary catalytic reduction reaction conditions, such as reaction with hydrogen gas at a temperature of room temperature to 100°C under acidic, neutral or alkaline conditions in the presence of a catalyst such as Pd-C. A method of treatment using zinc or tin under acidic conditions or a method of using zinc powder under neutral or alkaline conditions may also be employed.

The reaction between the orthophenylenediamine derivative (a2) and CS₂ may be conducted by, for example, the methods described in J. Org. Chem., 1954, Vol. 19, p.631-637 (pyridine solution) or.J. Med. Chem., 1993, Vol. 36, p.1175-1187 (ethanol solution).

The reaction between the thiobenzimidazole (a3) and halide ester (a4) may be conducted under ordinary S-alkylation conditions, and for example, by stirring at a temperature of 0°C-200°C in the presence of a base such as NaH, Et₃N, NaOH or K₂CO₃.

As halogenation reagents for conversion of the 3-hydroxymethyl-benzothiophene derivative (II) to (a6) there may be mentioned hydrogen halides, phosphorus halides, sulfonic acid chloride, thionyl halides and the like, among which phosphorus halides and thionyl halides are preferred, and phosphorus tribromide is especially preferred. As solvents there may be mentioned hydrocarbons such as cyclohexane and hexane and aromatic hydrocarbons such as benzene, toluene and xylene, among which cyclohexane and toluene are preferred. The reaction may be carried out from room temperature to reflux temperature, for 10 minutes or more to several hours.

The reaction between the thiobenzimidazole (a5) and halide derivative or ammonium salt (a6) may be conducted under ordinary N-alkylating or N-acylating conditions, and for example, by stirring at a temperature of 0°C-200°C in the presence of a base such as NaH, Et₃N, NaOH, K₂CO₃ or Cs₂CO₃.

As the dissociation reaction for the carboxyl protecting group R₂₅, there is preferably employed a method of hydrolysis using an alkali such as lithium hydroxide or an acid such as hydrochloric acid or trifluoroacetic acid.

### Synthesis Scheme (B):

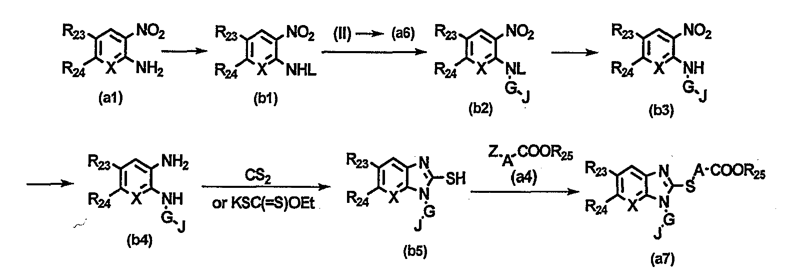

Specifically, the amino group of a 2-nitroaniline derivative (a1) is protected with an appropriate protecting group L to obtain (b1). This is reacted with a halide derivative (a6) obtained by halogenating the hydroxyl group of formula (II) to obtain (b2), and L is deprotected to obtain (b3). The nitro group of (b3) is reduced to obtain an orthophenylenediamine derivative (b4). This is then reacted with CS₂ or KSC(=S)OEt to form compound (b5), which may be reacted with a halide ester derivative (a4) to obtain benzimidazole derivative (a7). If necessary, this product may be further subjected to hydrolysis to obtain a benzimidazole derivative where R₂₅ is a hydrogen atom.

Alternatively, the halide derivative (a6) may be reacted without protection of the 2-nitroaniline derivative (a1) to directly obtain compound (b3). The protecting group L may be trifluoroacetyl, acetyl, t-butoxycarbonyl, benzyl or the like. The reaction between the orthophenylenediamine derivative (b4) and CS₂ may be conducted in the same manner as in Synthesis Scheme (A), and the reaction with KSC(=S)OEt may be carried out by the method described in Organic Synthesis (OS) 1963 Vol. 4, p.569-570. The other reactions may be carried out in the same manner as in Synthesis Scheme (A).

When E is tetrazol-5-yl and M is S in the benzimidazole derivative (XX), production may be carried out by Synthesis Scheme (E) shown below.

### Synthesis Scheme (E):

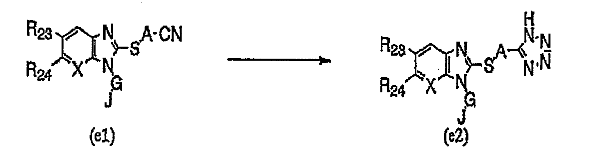

wherein R₂₃, R₂₄, A, G, J and X are as defined above.

A cyano compound (e1) is reacted with an azide for conversion to a tetrazole compound (e2). As azides there may be mentioned trialkyltin azide compounds such as trimethyltin azide, and hydroazic acid or its ammonium salt. When an organic tin azide compound is used, it is preferably used in about a 1-4 molar amount with respect to compound (e1). When hydroazic acid or its ammonium salt is used, sodium azide and ammonium chloride or a tertiary amine such as triethylamine, are preferably used in amount a 1-5 molar amount with respect to compound (e1). Each reaction is carried out using a solvent such as toluene, benzene or N,N-dimethylformamide at a temperature of 0°C-200°C.

When M is SO or SO₂ in the benzimidazole derivative (XX), production may be carried out by Synthesis Scheme (F) shown below.

### Synthesis Scheme (F):

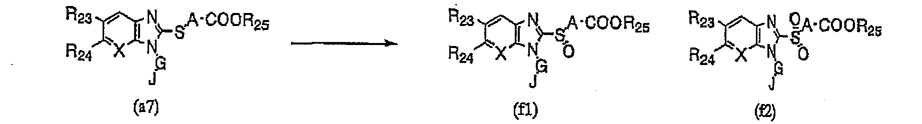

wherein R₂₃, R₂₄, R₂₅, A, G, J and X are as defined above.

Specifically, a benzimidazole compound (a7) is reacted with a peroxide compound in an appropriate solvent to obtain a sulfoxide derivative (f1) and/or sulfone derivative (f2). The peroxide compound used may be, for example, perbenzoic acid, m-chloroperbenzoic acid, peracetic acid, hydrogen peroxide or the like, and the solvent used may be, for example, chloroform, dichloromethane or the like. The proportion in which compound (a7) and the peroxide compound are used is not particularly restricted and may be selected as appropriate within a wide range, but for most purposes it is preferably between about a 1.2- and 5-molar amount. Each reaction is carried out at normally about 0-50°C and preferably between 0°C and room temperature, and is usually completed by about 4-20 hours.

When M in the benzimidazole derivative (XX) is a single bond, production may be carried out by Synthesis Scheme (G) shown below.

### Synthesis Scheme (G):

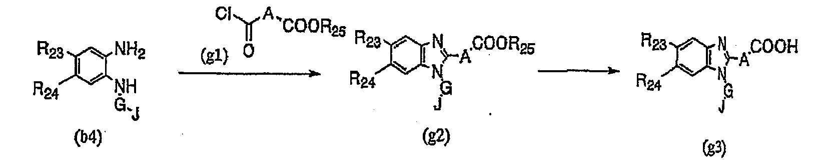

wherein X, A, G, J and R₂₅ are as defined above.

Specifically, a known acid chloride derivative (g1) is reacted with a diamine compound (b4) to obtain a benzimidazole derivative (g2). If necessary, the -COOR₂₅ group of (g2) may be hydrolyzed to obtain a benzimidazole derivative (g3) where R₂₅ is a hydrogen atom.

The cyclizing reaction is described in J. Med. Chem., 1993, Vol.36, p.1175-1187.

### Examples

The present invention will now be explained by the following examples, with the understanding that the invention is in no way limited by these examples.

### [Example 1]

### Synthesis of 3-chloro-4-methylbenzo[b]thiophene-2-carboxylic acid

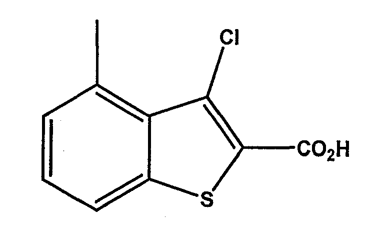

To a heated stirring mixture of 2-methylhydrocinnamic acid (20.00 g, 122 mmol), pyridine (1.0 mL, 12.2 mmol) and thionyl chloride (11.0 mL, 152 mmol) at 150°C there was slowly added dropwise thionyl chloride (32.0 mL, 396 mmol) at 150°C over a period of 2.5 hours, and stirring was continued for 3 hours. After 3 hours, the mixture was returned to room temperature, water (120 mL), 35% hydrochloric acid (12 mL) and tetrahydrofuran (200 mL) were added and the mixture was stirred at 60°C for 30 minutes. After 30 minutes, the tetrahydrofuran was distilled off under reduced pressure and the precipitated crystals were filtered out and dissolved in ethanol (100 mL) and water (300 mL) and stirred at 90°C for 1 hour, after which the solution was returned to room temperature and stirred overnight. The crystals that further precipitated were filtered out and recrystallized with a mixture of toluene (500 mL) and hexane (500 mL) to obtain 3-chloro-4-methylbenzo[b]thiophene-2-carboxylic acid (17.85 g, 78.7 mmol) (yield: 65%).
¹H-NMR (400 MHz, CDCl₃) δ(ppm) : 7.64(1H,d), 7.34(1H,t), 7.16(1H,d), 3.80(1H,br), 2.92(3H,s).

### [Example 2]

### Synthesis of sodium 3-chloro-4-methylbenzo[b]thiophene-2-carboxylate

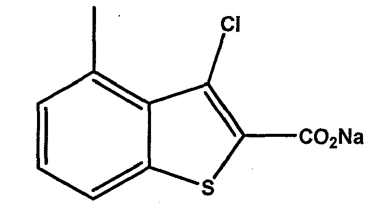

To a heated stirring mixture of 2-methylhydrocinnamic acid (36.00 g, 219 mmol), pyridine (1.9 mL, 23.5 mmol) and thionyl chloride (24.0 mL, 333 mmol) at 150°C there was slowly added dropwise thionyl chloride (55.0 mL, 763 mmol) at 150°C over a period of 2.5 hours, and stirring was continued for 3 hours. After 3 hours, the mixture was returned to room temperature, water (220 mL) and tetrahydrofuran (360 mL) were added and the mixture was stirred for 40 minutes. After 40 minutes, the tetrahydrofuran was distilled off under reduced pressure and extraction was performed with ethyl acetate (extraction with 360 mL followed by extraction with 180 mL), after which the extract was washed with water (180 mL, 3 times), sodium hydroxide (17.5 g, 438 mmol) dissolved in water (70 mL) was added and the precipitate crystals were filtered out and dried to obtain sodium 3-chloro-4-methylbenzo[b]thiophene-2-carboxylate (47.50 g) as a crude solid. The product was used without purification for the following reaction.

### [Example 3]

### Synthesis of 3-chloro-4-methylbenzo[b]thiophene

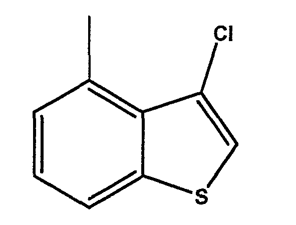

A mixture of 3-chloro-4-methylbenzo[b]thiophene-2-carboxylic acid (17.85 g, 78.7 mmol), copper (2.50 g, 39.3 mmol) and quinoline (179.0 mL, 1.51 mol) was stirred at 150°C for 3 hours. Upon completion of the reaction, the mixture was returned to room temperature, hexane (350 mL) was added and then the copper was removed by filtration and the filtrate was washed with 6M hydrochloric acid (350 mL), 1M hydrochloric acid (175 mL) and brine (175 mL) in that order. It was then dried over magnesium sulfate and the solvent was distilled off to obtain 3-chloro-4-methylberizo[b]thiophene (12.86 g, 70.4 mmol) (yield: 89.4%).
¹H-NMR (400 MHz, CDCl₃) δ(ppm): 7.64(1H,d), 7.25(1H,s), 7.23(1H,t), 7.12(1H,d), 2.87(3H,s).

### [Example 4]

### Synthesis of 3-chloro-4-methylbenzo[b]thiophene

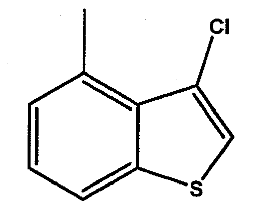

A mixture of sodium 3-chloro-4-methylbenzo[b]thiophene-2-carboxylate (47.5 g as crude solid), copper (3.65 g, 57.4 mmol) and N,N-dimethylacetamide (475 mL) was stirred at 160°C for 2 hours. Upon completion of the reaction, the mixture was returned to room temperature, hexane (950 mL) was added, and then the copper was removed by filtration and the filtrate was washed with 1M hydrochloric acid (washing first with 950 mL and then with 480 mL) and brine (480 mL) in that order. Silica gel (170 g) was then added and the mixture was stirred at room temperature for 30 minutes. The silica gel was filtered out and the filtrate was distilled off under reduced pressure to obtain 3-chloro-4-methylbenzo[b]thiophene (14.02 g, 76.8 mmol) (two-steps yield: 35.0%).

### [Example 5]

### Synthesis of ethyl 4-methylbenzo[b]thiophene-3-carboxylate

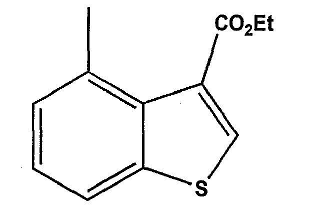

In a pressure-resistant glass container there were placed 3-chloro-4-methylbenzo[b]thiophene (5.0 g, 27.4 mmol), palladium acetate (307 mg, 1.37 mmol), sodium iodide (4.92 g, 32.8 mmol), 1,3-bis(diphenylphosphino)propane (594 mg, 1.44 mmol), dry ethanol (150 mL) and 1,8-diazabicyclo[5,4,0]undec-7-ene (4.85 g, 32.8 mmol), and the mixture was stirred at 130°C for 3 hours under carbon monoxide (0.6 MPa). Upon completion of the reaction, the palladium residue was removed by filtration and the filtrate was concentrated under reduced pressure. Water (50 mL) and toluene (50 mL) were added to the concentrated residue and the mixture was stirred, filtered with Celite and subjected to a separating procedure. The toluene layer was washed with 5% hydrochloric acid (50 mL), saturated aqueous sodium hydrogencarbonate (50 mL) and brine (50 mL) in that order and then dried over sodium sulfate, after which the filtrate was distilled off under reduced pressure. The residue was dissolved in ethyl acetate (25 mL) and silica gel (2.0 g) was added prior to stirring for 30 minutes. The silica gel was filtered out and the filtrate was distilled off under reduced pressure to obtain ethyl 4-methylbenzo[b]thiophene-3-carboxylate (5.36 g, 24.3 mmol) (yield: 89%).
¹H-NMR (400 MHz, CDCl₃) δ (ppm) : 8.01 (1H, s), 7.71(1H,d), 7.30(1H,t), 7.21(1H,d), 4.40(2H,q), 2.67(3H,s), 1.42 (3H,t).

### [Example 6]

### Synthesis of 3-cyano-4-methylbenzo[b]thiophene

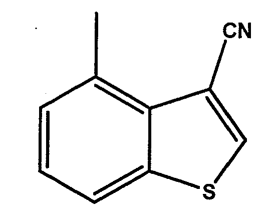

A mixture of nickel bromide (251.1 mg, 1.15 mmol), triphenylphosphine (1.21 g, 4.59 mmol), zinc (230 mg, 3.45 mmol) and ethanol (12 mL) was stirred at 88°C for 1.5 hours. After 1.5 hours, potassium cyanide (1.28 g, 19.69 mmol) and 3-chloro-4-methylbenzo[b]thiophene (3.0 g, 16.42 mmol) dissolved in ethanol (30 mL) were added in that order and stirring was continued for 3 hours. Upon completion of the reaction, the zinc was removed by filtration and the ethanol was distilled off, after which ethyl acetate (30 mL) was added and washing was performed with saturated aqueous sodium hydrogencarbonate (15 mL, twice), water (15 mL) and brine (15 mL). The mixture was then dried over magnesium sulfate and the solvent was distilled off to obtain 3-cyano-4-methylbenzo[b]thiophene (3.39 g) as a crude solid. The product was used without purification for the following reaction.
¹H-NMR (400 MHz, CDCl₃) δ(ppm): 8.12(1H,s), 7.72(1H,d), 7.33(1H,t), 7.22(1H,d), 2.88(3H,s).

### [Example 7]

### Synthesis of 4-methylbenzo[b]thiophene-3-carboxylic acid

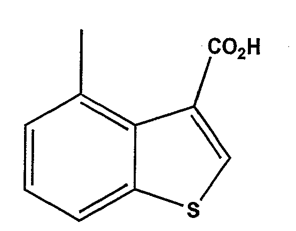

A mixture of 3-cyano-4-methylbenzo[b]thiophene (2.3 g as crude solid), sodium hydroxide (1.34 g, 33.5 mmol) and ethylene glycol (20 mL) was stirred at 160°C for 16 hours. Upon completion of the reaction, water (40 mL) was added and washing was performed with ethyl acetate (10 mL, twice), after which 6M hydrochloric acid (5.8 mL, 34.8 mmol) was added to the aqueous layer while cooling on ice and stirring was continued for awhile until precipitation of crystals. The crystals were filtered, washed with water (5 mL, twice) and dried to obtain 4-methylbenzo[b]thiophene-3-carboxylic acid (1.66 g, 8.63 mmol) (two-stage yield: 77.7%).
¹H-NMR (400 MHz, CDCl₃) δ (ppm): 8.12(1H,s), 7.71(1H,d), 7.29(1H,t), 7.22(1H,d), 3.74(1H,br), 2.71(3H,s).

### [Example 8]

### Synthesis of 3-hydroxymethyl-4-methylbenzo[b]thiophene

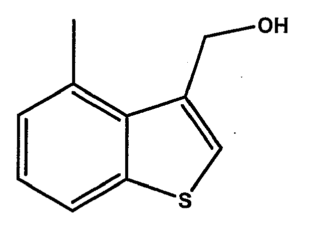

After dissolving 4-methylbenzo[b]thiophene-3-carboxylic acid (250.0 mg, 1.30 mmol) in tetrahydrofuran (3.0 mL), a borane-tetrahydrofuran complex (3.9 mL, 3.9 mmol) was slowly added dropwise over a period of 30 minutes while cooling on ice, and the mixture was stirred at room temperature for 9 hours. Upon completion of the reaction, 2M hydrochloric acid (2.5 mL) was slowly added for quenching, and then ethyl acetate (40 mL) was added and the mixture was washed with water (15 mL) and brine (15 mL) in that order, after which it was dried over magnesium sulfate and the solvent was distilled off to obtain 3-hydroxymethyl-4-methylbenzo[b]thiophene (232.3 mg, 1.30 mmol) (yield: 100%).
¹H-NMR (400 MHz, CDCl₃) δ(ppm): 7.70(1H,d), 7.42(1H,s), 7.24(1H,t), 7.14(1H,d), 5.03(2H,s), 2.81(3H,s).

### [Example 9]

### Synthesis of 3-hydroxymethyl-4-methylbenzo[b]thiophene

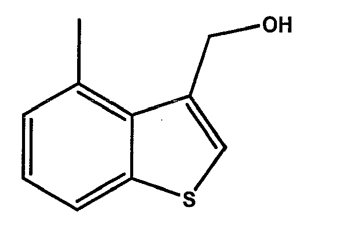

After dissolving ethyl 4-methylbenzo[b]thiophene-3-carboxylate (37.0 mg, 0.168 mmol) in tetrahydrofuran (0.5 mL), lithium aluminum hydride (7.6 mg, 0.20 mmol) was added while cooling on ice and the mixture was stirred for 45 minutes. Upon completion of the reaction, 2M hydrochloric acid (0.1 mL) was slowly added for quenching, and then ethyl acetate (10 mL) was added and the mixture was washed with water (5 mL) and brine (5 mL) in that order, after which it was dried over magnesium sulfate and the solvent was distilled off to obtain 3-hydroxymethyl-4-methylbenzo[b]thiophene (28.7 mg, 0.161 mmol) (yield: 96%).

### Industrial Applicability

The 3-hydroxymethyl-4-methylbenzo[b]thiophenes obtained by the production process of the invention can be used as production intermediates for drugs such as chymase inhibitors.

## Claims

1. A production process in which a compound represented by the following formula (I): 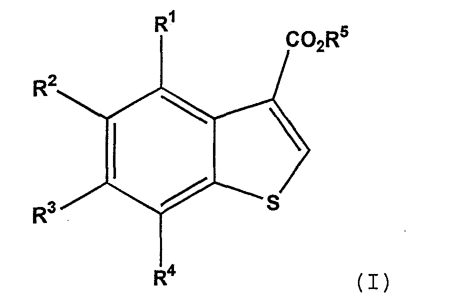 wherein
R¹ represents C1-6 alkyl, C1-6 halogenated alkyl, a halogen atom, cyano, C1-6 alkoxy, C1-6 alkylthio, C1-6 acyloxy, C1-6 acylamino or C1-6 halogenated alkoxy; and
R², R³ and R⁴ simultaneously or each independently represent hydrogen, C1-6 alkyl, C1-6 halogenated alkyl, a halogen atom, cyano, C1-6 alkoxy, C1-6 alkylthio, C1-6 acyloxy, C1-6 acylamino or C1-6 halogenated alkoxy; and
R⁵ represents hydrogen or a C1-6 alkyl group;
is reduced to obtain a 3-hydroxymethylbenzo[b]thiophene derivative represented by the following formula (II): 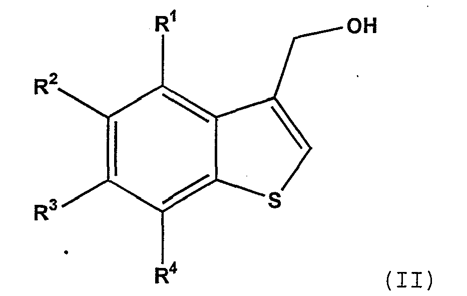 wherein R¹, R², R³ and R⁴ are as defined in formula (I).

2. The production process according to claim 1, wherein a metal hydride complex is used as the reducing agent.

3. The production process according to claim 2, wherein the metal hydride complex is sodium bis(2-methoxyethoxy)aluminum hydride.

4. The production process according to any one of claims 1 to 3, wherein a compound represented by the following formula (III) is subjected to hydrolysis to produce a benzo[b]thiophene derivative represented by formula (I): 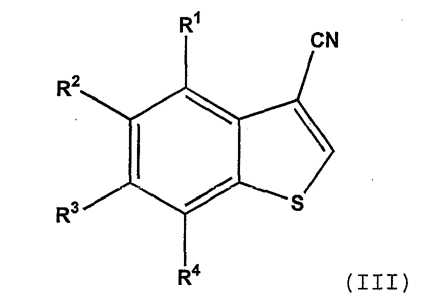 wherein R¹, R², R³ and R⁴ are as defined in formula (I).

5. The production process according to claim 4, wherein the hydrolysis is basic hydrolysis.

6. The production process according to claim 5, **characterized in that** the basic hydrolysis employs sodium hydroxide.

7. The production process according to any one of claims 4 to 6, wherein the chloro group of a compound represented by the following formula (IV): 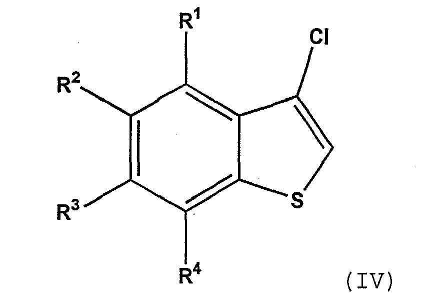 wherein R¹, R², R³ and R⁴ are as defined in formula (I);
is substituted with a cyano group, to produce the benzo[b]thiophene derivative represented by formula (III).

8. The production process according to claim 7, wherein potassium cyanide, sodium cyanide, copper cyanide or zinc cyanide is used to substitute the chloro group of the compound represented by formula (IV) with a cyano group.

9. The production process according to claim 8, wherein a nickel catalyst is used.

10. The production process according to any one of claims 1 to 3, wherein the chloro group of a compound represented by formula (IV) is substituted with a carboxyl group, to produce a benzo[b]thiophene derivative represented by formula (I).

11. The production process according to claim 10, **characterized by** using carbon monoxide.

12. The production process according to claim 11, **characterized by** using a palladium catalyst.

13. The production process according to any one of claims 7 to 12, wherein a benzo[b]thiophene derivative represented by formula (IV) is produced by decarboxylation of a compound represented by the following formula (V): 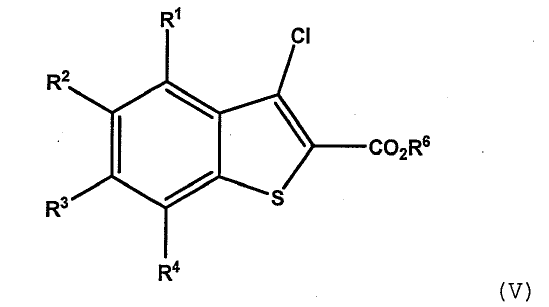 wherein R¹, R², R³ and R⁴ are as defined in formula (I), and R⁶ represents a hydrogen atom or a metal.

14. The production process according to claim 13, wherein a benzo[b]thiophene derivative represented by formula (V) is produced by reaction of a compound represented by the following formula (VI): 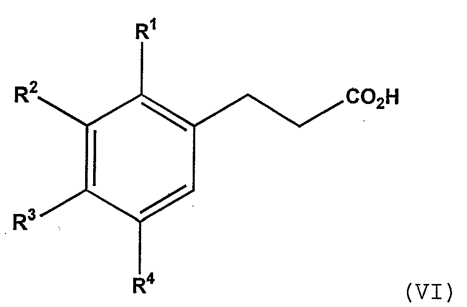 wherein R¹, R², R³ and R⁴ are as defined in formula (I); with thionyl chloride for cyclization.

15. The production process according to claim 13 or 14, wherein R⁶ in formula (V) is an alkali metal.

16. The production process according to claim 15, wherein R⁶ in formula (V) is a sodium atom.

17. A production process for benzimidazole derivatives represented by formula (XX), which includes the following production steps:
(i) a production process for a compound represented by formula (II), according to any one of claims 1 to 16; and
(ii) a process in which a benzimidazole derivative represented by formula (XX) is produced from the compound represented by formula (II); 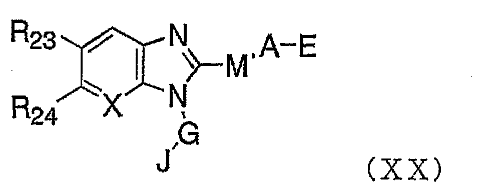 in formula (XX),
R₂₃ and R₂₄ simultaneously or each independently represent a hydrogen atom, a halogen atom, trihalomethyl, cyano, hydroxyl, C1-4 alkyl or C1-4 alkoxy, or R₂₃ and R₂₄ may together form -O-CH₂O-, -O-CH₂CH₂O- or -CH₂CH₂CH₂- (in which case the carbon atoms may be optionally substituted with one or more C1-4 alkyl groups);
A represents a substituted or unsubstituted C1-7 straight-chain, cyclic or branched alkylene group or alkenylene group, which may include one or more group(s) selected from the group consisting of -O-, -S-, -SO₂- and -NR₂₅- (where R₂₅ represents a hydrogen atom or a straight-chain or branched C1-6 alkyl group), and the substituent(s) on the groups may be a halogen atom or hydroxyl, nitro, cyano, straight-chain or branched C1-6 alkyl or straight-chain or branched C1-6 alkoxy groups (including cases where two adjacent groups form an acetal bond), straight-chain or branched C1-6 alkylthio, straight-chain or branched C1-6 alkylsulfonyl, straight-chain or branched C1-6 acyl, straight-chain or branched C1-6 acylamino, trihalomethyl, trihalomethoxy, phenyl, oxo or phenoxy optionally substituted with one or more halogen atom(s), and one or more of the substituents at any desired position of the alkylene or alkenylene group may be independently substituted, with the exclusion of cases where M in the formula is a single bond, and carbons of A bonded to M are simultaneously substituted with hydroxyl and phenyl groups;
E represents -COOR₂₅, -SO₃R₂₅, -CONHR₂₅, -SO₂NHR₂₅, tetrazol-5-yl, 5-oxo-1,2,4-oxadiazol-3-yl or 5-oxo-1,2,4-thiadiazol-3-yl (where R₂₅ is as defined above);
M represents a single bond or -S(O)m-, where m is an integer of 0-2;
G and J both represent formula (II) above, with G representing the methylene group at position 3 of the benzothiophene of formula (II), and the hydroxyl group of formula (II) is substituted for the nitrogen atom on the benzimidazole ring; and
X represents -CH= or a nitrogen atom.

18. A compound represented by the following formula (I) : 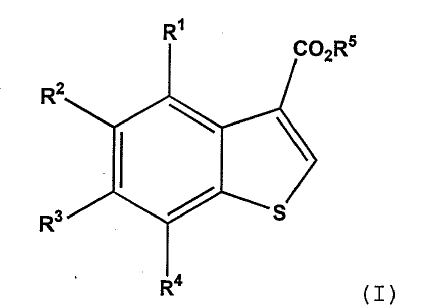 wherein R¹, R², R³, R⁴ and R⁵ are as defined above.

19. The compound according to claim 18, wherein R², R³ and R⁴ are hydrogen atoms.

20. The compound according to claim 18 or 19 wherein R¹ is a methyl group.

21. The compound according to any one of claims 18 to 20, wherein R⁵ is an ethyl group or a hydrogen atom.
